# EUROPEAN PATENT APPLICATION

(11) **EP 3 939 621 A1**
(43) Date of publication of application: **19.01.2022**
(21) Application number: 20382648.2
(22) Date of filing: 17.07.2020
(51) Int. Cl.: A61L 2/10, A61L 2/28

(54) **DISINFECTION-STERILIZATION SYSTEM AND PROCEDURE**

(71) Applicant: Asti Mobile Robotics, S.A.U., 09390 Madrigalejo Del Monte (Burgos) (ES); Boos Technical Lighting, S.L., 47155 Santovenia De Pisuerga (Valladolid) (ES)
(72) Inventor: CASCAJAR ORDOÑEZ, Luis, 09390 Madrigalejo del Monte (Burgos) (ES); MEDINA MERINO, José Manuel, 09390 Madrigalejo del Monte (Burgos) (ES); RODRÍGUEZ MERINO, Fernando, 47155 Santovenia De Pisuerga (Valladolid) (ES); SAN MIGUEL DE JUANA, Julián, 47155 Santovenia De Pisuerga (Valladolid) (ES)
(74) Representative: Pons

(57) **Abstract**

The system is applicable to the disinfection of different areas of buildings, such as hospitals, from pathogens and incorporates a robot (1) with high power ultraviolet UV-C radiation lamps (4) of vertical configuration, which are distributed over a circumference at the edge of the platform (3) in the location that corresponds to the direction of travel of the robot, a central unit (6) that emits and receives information to / from the robot (1) in which the path to be taken by the robot is stored and simulated (1), and UV-C radiation sensors (7) located within the area to be disinfected that record the radiation that reaches them during the disinfection process. The disinfection procedure includes the continuous movement of the robot during the application of UV-C ultraviolet radiation to avoid the interposition of shadows between the surface to be disinfected and the lamps.

## Description

### OBJECT OF THE INVENTION

The present invention is applicable to the disinfection of different areas of buildings, such as hospitals, of pathogens, for instance viruses and bacteria.

The disinfection-sterilization system, which is the objective of this invention, utilizes a robot with UV-C ultraviolet germicidal lamps and describes a method of disinfection-sterilization of both horizontal and vertical surfaces, which is based on the use of specially configured lamps, and on the continuous movement of the robot which avoids shadow interposition between the lamps and the surfaces to be disinfected.

### BACKGROUND OF THE INVENTION

The germicidal function of electromagnetic radiation in the ultraviolet-C range is well known, which is, between 200 nm to 300 nm. The lamps used for this purpose emit radiation primarily in the 254 nm range, which is a wavelength closer to the point of maximum radiation absorption by genetic material.

The level of disinfection achieved in the process for different pathogens, including bacteria and viruses, such as coronavirus, is intimately related to the amount of radiation that strikes the surfaces which are to be sanitized. The amount of radiation needed for each pathogen and for each level of disinfection is scientifically documented in multiple studies published in scientific journals.

The amount of radiation that reaches a surface mainly depends on two factors: the distances from the surface to the source of radiation and the orientation of the surface with respect to the radiation source.

With regards to vertical surfaces, the entire length of the lamp influences the irradiation of the point to be carefully examined, and in the case of horizontal surfaces, only the part of the lamp that is above the horizontal surface contributes to the level of radiation upon said surface, as when radiating from a fixed point, any item of furniture or object generates a shadow. The germicidal effect does not occur on shadow points, since they are not irradiated by UV-C energy, and therefore disinfection does not occur.

Disinfection robots are known to be utilized which employ UV lamps to disinfect, work discontinuously, and stop before each surface, dedicating a significant amount of time to apply a greater amount of radiation in order to disinfect those surfaces where shadows interpose.

### DESCRIPTION OF THE INVENTION

The disinfection system proposed by the present invention basically comprises a robot that incorporates vertically configured high-power UV-C ultraviolet lamps, a central unit that emits and receives information to / from the robot that is stored and simulated. The information stored in this unit includes the path to be followed by the robot within the rooms of the area to be irradiated where the disinfection of the surfaces is carried out, both of the area and of the physical elements located within that area. There are also sensors located within the area that record UV-C radiation that reaches them during the disinfection process.

The system is developed with a configuration and continuous operation mode of the robot, which allows the UV-C radiation lamps to be brought as close as possible to the surfaces that need to be irradiated, whether they are vertical or horizontal surfaces, avoiding, by way of this continuous movement, the interposition of shadows between the surface to be disinfected and the lamps.

The robot comprises a drive unit, a platform mounted on that drive unit, the lamps, preferably 4 in number, which are distributed on a circumference at the edge of the platform at the location which corresponds to the direction of travel of the robot, and a control unit incorporating wireless communication, guidance, and navigation means.

In the case of using 4 lamps, two on each end and two central, the lamps are located on one side of the centre of the aforementioned circumference in a radial arrangement and equidistant from the centre of said circumference, separated from each other by 45º, where the lamps on either end each form an angle of 22.5º with respect to the median plane of the circumference.

The robot lamps are preferably 1600 mm high and, together with the drive unit, have a height of approximately 2 meters. This configuration allows for an optimal level of radiation on horizontal surfaces that are above 1.5 meters (billing desks, hospital reception desks, etc.). In the same way, this configuration allows for a homogeneous irradiation of the walls from the ground up to 2.5 meters high.

With regard to each of the lamps, there is a reflector located behind the lamp that increases the amount of radiation emitted forward. The reflector is preferably made of a pre-anodized aluminium specific for ultraviolet radiation reflection. The reflector's geometry allows the UV-C light to be projected forward and to the sides of the robot.

The incorporation of high-power lamps, reflectors, and their distribution on the platform, delivers an optimal radiation level at each point on the horizontal and vertical surfaces without the need for the robot to make stops to reach this value.

The continuous movement disinfection process allows the elimination of shadows. By equipping the robot with a powerful enough UV-C energy emission source, the process can be carried out continuously, covering linear movement speeds of between 50 mm per second to 500 mm per second and angular speeds of 1 degree per second up to 20 degrees per second.

Within these linear and angular speed ranges, the UV-C energy level necessary to reach different degrees of disinfection (D90, D99, D99.9, D99.99, etc.) is achieved when dealing with different pathogens such as viruses, bacteria, protozoa and spores.

The elongated geometry of the lamps, the arrangement of their UV-C light emitting tubes, together with the reflectors and the continuous movement of the robot, avoid macroscopic shadows owing to objects and microscopic shadows due to the intrinsic roughness of materials where viruses and bacteria may have been deposited.

Regarding the disinfection procedure, the process is carried out in accordance with the following stages:
- the study of the area to be disinfected. Computer simulation of said area in order to determine what is the optimum path for disinfection. This simulation allows one to know the radiation values of the different surfaces and to optimize the robot's movement parameters (speed, distances, trajectories) to achieve the optimum result,
- the receipt by the robot control unit of information from the central unit relative to the path to be carried out within an area to be disinfected, including parameters of movement: linear speed of travel, angular speed within the path, time, and radiation intensities to be applied to the trajectory,
- the continuous movement of the robot, managed by the control unit, at the established travel speeds, along the trajectory within the area to be disinfected,
- the application of ultraviolet UV-C radiation during the movement of the robot continuously along the path, using the lamps at the established intensity,
- the capture by the sensors of the radiation values received within the area and the transmission of these values to the robot,
- the transmission, by the robot control unit to the central unit, of information regarding its position and trajectory, and the radiation values captured by the sensors.

The information regarding the trajectory to be performed is obtained through a computer simulation which includes the following steps:
- the input of data related to the geometry of the surfaces of the area to be disinfected and of the physical elements, such as tables, chairs, beds, etc., that are within the area to be disinfected,
- the calculation of the level of radiation reaching each surface based on movement parameters: linear speed and angular velocity of the robot, distance to different surfaces, path of the robot, and verification of the shadows produced by each surface,
- the verification of the recommended UV-C radiation dose depending on the pathogen and the level of disinfection intended,
- the generation of movement and time parameters needed to achieve the radiation level necessary for the pathogen and disinfection level desired upon each surface.

The verification of the recommended dose of UV-C radiation depending on the pathogen and the level of disinfection intended can be carried out with the aid of scientific databases published in scientific journals.

It should be noted that during the disinfection process, the robot follows the route obtained in the simulation process using its guidance and navigation system. In the same way, it records its position relative to the different elements of the room so that there is a traceability record of where and how the robot has moved at all times. This is due to the fact that the room may not be exactly the same as originally planned and the robot has had to change its path in order to avoid objects.

The actual trajectory recorded by the robot is stored for each and every one of the disinfection processes and can be used to recalculate, using the simulation software, the levels obtained on all surfaces in the actual displacement during each of the actions.

Traceability information and simulations for actual trajectories can be used to carry out audit reports on disinfection processes.

Additionally, the values recorded by the UV-C measurement sensor are compared with the value obtained in the simulation for the same point where the sensor was placed, and they are stored in the central unit.

The comparison of both values, real and simulated, allows the system to readjust the robot's displacement parameters to achieve the optimal disinfection values indicated.

This optimization of the navigation path is sent wirelessly to the robot so as the following time the disinfection process is carried out in the same room, it is done in the most efficient way possible.

The sensors used are UV-C recorders calibrated for 254 nm and are connected to the robot through a Bluetooth (wireless) connection. Every second, they communicate what the level of UVC 254 nm radiation is that is reaching them.

The method of operation is such that, when the robot enters a room, it checks if there is any sensor within it by studying the room configuration documentation in the central unit. If so, once this verification has been confirmed, an attempt is made to connect wirelessly with this sensor. Each sensor has a unique identification code.

Once the connection is established, the robot begins to continuously receive the UV-C radiation levels that the sensor registers. Aforementioned values are stored in the memory of the robot control unit, in such a way that, once the disinfection process has finished, the robot has real information on what has been the accumulated level of radiation during the entire process at one point in the room.

The spatial coordinates where the sensor(s) is(are) located, as there may be several working at once, and the orientation of the measuring head provided by the sensor, are known, and recorded in the central unit.

The information recorded in the disinfection process by the sensors, based on the fact that the position of the sensors is perfectly known, is used to carry out a continuous auditing process of the disinfection procedure, since the data obtained is compared with the values given in the simulations of said room.

This auditing process is complemented by the traceability information of the robot that delivers the position, orientation, and speed of the robot at all times within the room.

The actual measurements are compared with the simulations and from the comparative analysis of these two sources of results, actions are taken to improve the disinfection process.

### DESCRIPTION OF THE DRAWINGS

To enhance the description that is being made and to aid a better understanding of the characteristics of the invention, in accordance with a preferred example of practical embodiment thereof, a set of drawings is included as an integral part of said description, where, by way of illustration and not limitation, the following has been represented:
Figure 1.- Shows a view of the disinfection-sterilization system that constitutes the objective of this invention.
Figure 2.- Shows a top view of the details of the relative location of the lamps and reflectors on the robot.

### PREFERRED EMBODIMENT OF THE INVENTION

Below, regarding the figures, a mode of preferred embodiment of the disinfection-sterilization system is described.

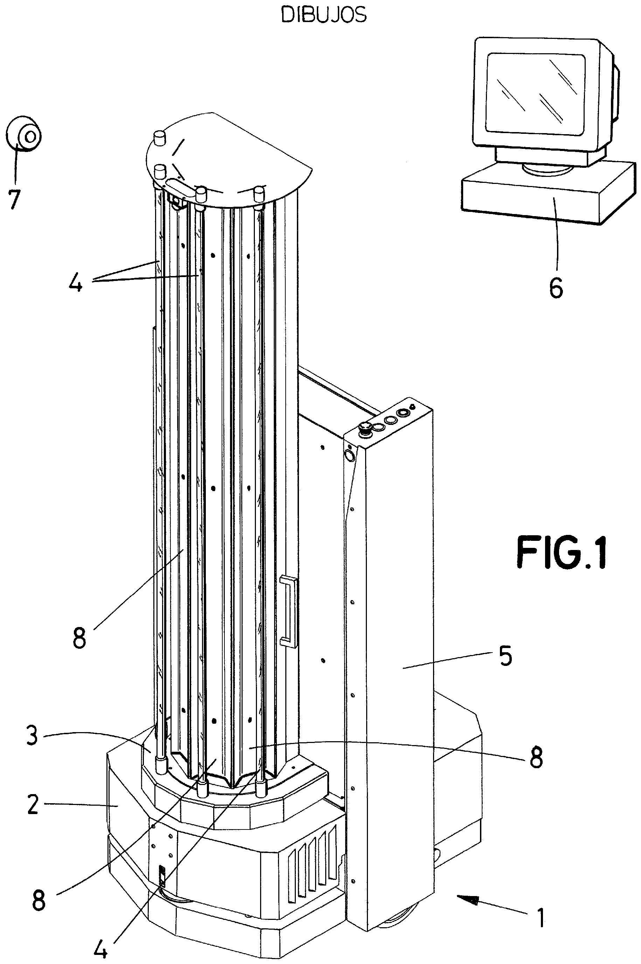

As seen in figure 1, the system comprises a robot (1) equipped with a drive unit (2), a platform (3) mounted on that drive unit (2), four UV-C ultraviolet radiation lamps (4) located on the platform (3), and a control unit (5) that incorporates wireless communication, guidance and navigation means.

Additionally, the system comprises a central unit (6) that emits and receives information to / from the robot (1) in which the trajectory to be carried out by the robot (1) is stored and simulated, as well as including sensors (7) located within the area to be disinfected that record the UV-C radiation that reaches it during the disinfection process.

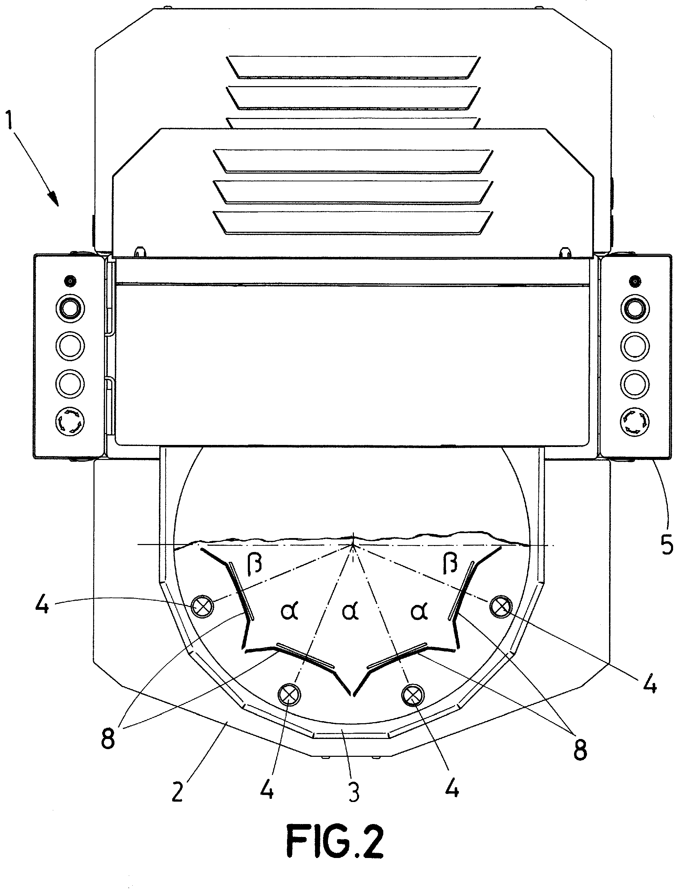

Figure 2 shows that the four lamps are distributed on a circumference, on the edge of the platform (3) in the location that corresponds with the direction of travel of the robot (1), distributed more specifically on one side of half of said circumference in a radial arrangement and equidistant from the centre of said circumference, separated from each other by an angle of α = 45°, where the end lamps each form an angle of β = 22.5° with respect to the median plane of the circumference.

Also, in Figure 2, it can be discerned that the robot (1) incorporates several reflectors as well (8), located behind each of the lamps (4), which increase the amount of radiation emitted forward.

## Claims

1. Disinfection-sterilization system intended for disinfection / sterilization of the surface of an area to be disinfected, and of the physical elements located within the area, **characterized in that** said system comprises:
a robot (1) which in turn comprises:
a drive unit (2),
a platform (3) mounted on the drive unit (2),
high-powered vertical configuration UV-C ultraviolet radiation lamps (4), which are distributed over a circumference at the edge of the platform (3) at the location that corresponds to the direction of travel of the robot,
reflectors (8) located behind each of the lamps (4) that increase the amount of radiation emitted, and
a control unit (5) incorporating wireless communication, guidance, and navigation means,
a central unit (6) that emits and receives information to / from the robot (1) in which the path to be carried out by the robot is stored and simulated (1), and UV-C radiation sensors (7) located within the area to disinfect which records the UV-C radiation that reaches it during the disinfection process.

2. The system of claim 1 wherein the lamps (4) are four lamps which are distributed on one side of the middle of the circumference in a radial arrangement and equidistant from the centre of said circumference, separated from each other by an angle of α = 45 °, where the lamps on either end each form an angle of β = 22.5° with respect to the median plane of the circumference.

3. The system 1 of claim 1, wherein the lamps (4) have a height of about 1600 mm.

4. The system of claim 2 in which the sum of the height of the drive unit (2) and that of the lamps (4) is approximately 2 meters.

5. Disinfection-sterilization procedure that makes use of the system described in any one of claims 1-4, **characterized by** the fact that it is carried out according to the following steps:
- the receipt by the control unit (5) of the robot (1) of information from the central unit (6) regarding the trajectory to be carried out within an area to be disinfected, including movement parameters: linear speed rate, speed angle, time, and radiation intensities to be applied upon the path,
- the continuous movement of the robot (1), managed by the control unit (5), at the established travel speeds, along the trajectory of the area to be disinfected,
- the application of ultraviolet UV-C radiation during the movement of the robot (1) continuously along its path, using the lamps (4) at the established intensity,
- the capture by the UV-C radiation sensors (7) of the radiation values received in the area and the sending of these values to the robot (1),
- the sending by the control unit (5) of the robot (1) to the central unit (6), with information regarding the position and trajectory carried out by the robot, and the radiation values captured by the sensors (7),
where the information regarding the trajectory to be performed is obtained by computer simulation, and comprises the steps of:
- the input of data related to the surface geometry of the area to be disinfected and the physical elements found in the area to be disinfected,
- the calculation of the level of radiation reaching each surface based on motion parameters: linear velocity and angular velocity of the robot, distance to different surfaces, robot path, and verification of the shadows produced by each surface,
- the verification of the recommended UV-C radiation dose depending on the pathogen and the level of disinfection intended,
- the generation of the necessary movement and time parameters to achieve the radiation level needed for the pathogen, and disinfection level desired on each surface.
